# EUROPÄISCHE PATENTSCHRIFT

(11) **EP 2 548 522 B1**
(45) Veröffentlichungstag und Bekanntmachung des Hinweises auf die Patenterteilung: **04.10.2017**
(21) Anmeldenummer: 12177123.2
(22) Anmeldetag: 19.07.2012
(51) Int. Cl.: A61B 17/17

(54) **Vorrichtung zum Anzielen und Einbringen von mehreren Bohrkanälen in einen Knochen**
Device for targeting and inserting multiple drilled channels in a bone
Dispositif de visée et d'insertion de plusieurs canaux de perçage dans un os

(30) Priorität: 22.07.2011 DE 102011108673
(43) Veröffentlichungstag der Anmeldung: 23.01.2013
(73) Patentinhaber: Karl Storz GmbH & Co. KG, 78532 Tuttlingen (DE)
(72) Erfinder: Berberich, Sascha, 78532 Tuttlingen (DE); Weinmann, Daniel, 78606 Seitingen- Oberflacht (DE)
(74) Vertreter: Witte, Weller & Partner Patentanwälte mbB

(56) Entgegenhaltungen:
- WO-A1-2006/056754
- DE-U1- 8 411 993
- FR-A1- 2 918 554
- US-A- 5 324 295

## Beschreibung

Die Erfindung betrifft eine Vorrichtung zum Anzielen und Einbringen von mehreren Bohrkanälen in einen Knochen, mit einer Handhabe, in der abnehmbar eine einzige Führungshülse aufgenommen ist, in der ein mittiger erster Führungskanal für einen ersten Zieldraht vorhanden ist, wobei die Führungshülse über deren distales Ende an einen Knochen anlegbar ist, und mit einem von der Handhabe vorstehenden Arm, dessen distales Ende an einer weiteren Stelle am Knochen anlegbar ist, wobei die Führungshülse einen zweiten Führungskanal für einen weiteren Führungsdraht aufweist, und eine Mittellängsachse des zweiten Führungskanals in einem Abstand zur Mittellängsachse des ersten Führungskanals und außerdem parallel zu diesem verläuft.

Eine derartige Vorrichtung ist aus der DE 84 11 993 U1 bekannt.

Die US 5 324 295 A betrifft eine Vorrichtung zum Führen von zwei Bohrdrähten an eine bestimmte Stelle in einem Kniegelenk. Konvergierende Führungskanäle erlauben es die Bohrdrähte von unterschiedlichen Stellen aus anzusetzen, die jedoch konvergierend auf dieselbe Stelle geführt werden. In die konvergierenden Führungskanäle können Einsätze eingebracht werden, die mehrere, parallel zueinander verlaufende Führungsbohrungen aufweisen, so dass durch die Einsätze mehrere, parallel zueinander verlaufende Bohraugen gesetzt werden können. Ein Einsatz, durch den bereits ein Bohrdraht hindurchgeführt ist, kann um diesen Bohrdraht gedreht werden, so dass im Falle eines schlecht gesetzten Bohrdrahtes ein parallel dazu verlaufender besser plazierter Bohrdraht gesetzt werden kann.

Derartige Zielgeräte dienen dazu, Bohrkanäle in einem Knochen einzubringen, wobei in die Bohrkanäle Implantate eingesetzt werden.

Ein weit verbreiteter Einsatzbereich ist die Rekonstruktion von Kreuzbändern im Bereich des Knies. Das vordere Kreuzband erstreckt sich vom oberen Plateau (Tibiaplateau) des Unterschenkels (Tibia) und verläuft zur Innenseite des unteren Endes des Oberschenkelknochens (Femur).

Bei schweren Verletzungen wird das Kreuzband vollständig entfernt und durch ein Sehnenimplantat ersetzt.

Oftmals ist dieses Sehnenimplantat eine Sehne des Patienten selbst, z.B. die Semitendinosussehne, die sich als besonders nützlich für die Rekonstruktion von Kreuzbändern im Knie erwiesen hat.

Um eine möglichst naturgetreue Ausrichtung des Sehnenimplantates zu erzielen, muss dieses in dem Knochen festsitzend verankert werden. Dazu müssen in den Knochen entsprechende Bohrungen eingebracht werden.

Da, wie zuvor erwähnt, sich das Kreuzband in einer ganz bestimmten Richtung vom Unterschenkelknochen zum Oberschenkelknochen erstreckt, sollten sich die Bohrungen in diesen Knochen, in die das Sehnenimplantat eingesetzt und verankert werden soll, möglichst ebenfalls in dieser Richtung erstrecken.

Dadurch kann erzielt werden, dass sich das Sehnenimplantat von der Knochenoberfläche, bspw. vom Tibiaplateau aus der Verankerungsstelle herausreichend, bereits in die anatomisch richtige Richtung erstreckt.

Dazu müssen exakt ausgerichtete Bohrungen in dem Knochen durchgeführt werden.

Da solche operativen Eingriffe arthroskopisch durchgeführt werden und das Kniegelenk ein kompliziertes und enges Gelenk darstellt, steht dem Operateur relativ wenig Raum zur Verfügung, um solche Bohrungen zielgerecht anzusetzen und durchzuführen.

Dazu haben sich die eingangs erwähnten Vorrichtungen zum Anzielen und Einbringen von Bohrkanälen bewährt.

Bei der eingangs genannten Vorrichtung ist an der Handhabe, die stab- bzw. griffartig ausgebildet ist, eine einzige Führungshülse aufgenommen, durch die mehrere so genannte Zieldrähte hindurchgeschoben werden können. Solche Zieldrähte sind relativ steife, angespitzte Metalldrähte mit einem Durchmesser im Bereich von 2 bis 3 mm.

Bei der Handhabung wird z.B. das distale, relativ schmale Ende des von der Vorrichtung abstehenden Armes in das Kniegelenk eingeschoben und am Tibiaplateau angesetzt. Das distale Ende der beweglichen Führungshülse wird meist schräg an eine Außenseite des Unterschenkels angesetzt.

Bei der Rekonstruktion von Bändern, insbesondere solche, die sich in Bündel unterschiedlicher Richtungen erstrecken, werden Sehnenimplantate eingesetzt, die keinen kreisförmigen Querschnitt aufweisen. Das liegt bspw. daran, dass ein Sehnenband einer bestimmten Länge zu einer U-förmigen Schleife mit aneinanderliegenden Schenkeln des U verwendet wird. Ein solcher Doppelstrang hat im Querschnitt die Form einer "8", mit einer groben Hüllkurve versehen, eine etwa rechteckige Form.

Es werden auch Transplantate mit einem eckigen Knockenblock, z.B. Patella mit Patellasehne, oder Quadrizepssehnen eingesetzt.

Es besteht daher ein Bedarf an Vorrichtungen, mit denen von der Kreisgeometrie abweichende Bohrungen, insbesondere mehreckige und rechteckige Bohrungen vorbereitet und hergestellt werden können.

Solche Vorrichtungen sind beispielsweise aus der US 2006/0161163 A1, der US 2008/0097453 A1 und der US 6 022 356 A bekannt.

Es ist daher Aufgabe der vorliegenden Erfindung, eine Vorrichtung der eingangs genannten Art dementsprechend weiterzuentwickeln, so dass mehrere nebeneinander liegende Bohrkanäle bewerkstelligt werden können, die dann zu entsprechenden, von der runden Geometrie abweichenden Kanälen bearbeitet werden können.

Erfindungsgemäß wird die Aufgabe dadurch gelöst, dass an der Führungshülse zumindest eine Kanülierung vorhanden ist, deren Mittellängsachse im gleichen Abstand und parallel zur Mittellängsachse des ersten Führungskanals verläuft, und dass die zumindest eine Kanülierung umfangswinkelversetzt zur Mittellängsachse des zweiten Kanals verläuft, so dass ein durch den zweiten Führungskanal eingebrachter weiterer Zieldraht durch Abziehen, Drehen um den ersten Zieldraht und Wiedereinsetzen der Führungshülse in die zumindest eine Kanülierung einbringbar ist.

Diese Maßnahmen haben nun zahlreiche Vorteile.

Durch den zweiten Führungskanal, der in einem Abstand A und parallel zur Mittellängsachse des ersten Führungskanals verläuft, wird die grundsätzliche Voraussetzung geschaffen, zwei nebeneinander liegende und parallel zueinander verlaufende Zieldrähte zu setzen. Dabei kann der Abstand A so gewählt werden, dass nach Überbohren der Zieldrähte sich die beiden resultierenden kreisförmigen Bohrungen überschneiden, so dass hiermit grundsätzlich ein Kanal bewerkstelligt werden kann, der schon grob ein rechteckiges Umfangsprofil aufweist.

Durch Vorsehen der Kanülierung an der Außenseite der Führungshülse, die, was ihre Mittellängsachse betrifft, ebenfalls in dem Abstand A zur Mittellängsachse des mittigen Kanals verläuft und sich ebenfalls parallel zu dieser Achse erstreckt, können folgende Vorgänge eröffnet werden.

Nach Setzen der ersten beiden Zieldrähte kann die Führungshülse so weit abgezogen werden, dass diese sich nur noch auf dem mittleren, entsprechend längeren ersten Zieldraht befindet, den zweiten Führungsdraht aber nicht mehr aufnimmt. Somit ist der zweite Zieldraht aus dem zweiten Führungskanal ausgetreten. Nunmehr kann die Führungshülse um einen entsprechenden Winkel um den ersten Zieldraht gedreht werden, bis der zweite Zieldraht mit der Kanülierung in der Außenseite an der Führungshülse fluchtet. Durch Vorschieben der Führungshülse nach distal wird der zweite bzw. der zuletzt gesetzte Führungsdraht in die Kanülierung eingeschoben. Da in diesem Zustand der zweite Führungskanal nicht mehr belegt ist, kann durch diesen ein dritter Zieldraht hindurchgeschoben und in den Knochen eingebracht werden.

Je nach Anzahl dieser Kanülierungen und der Umfangswinkelorientierung können neben den beiden Zieldrähten, die durch den ersten und den zweiten Führungskanal gesetzt worden sind, nun noch weitere Zieldrähte gesetzt werden. Ist nur eine solche Kanülierung vorhanden, und liegt diese bspw. dem zweiten Führungskanal diametral gegenüber, können drei parallel zueinander verlaufende Führungsdrähte gesetzt werden. Nach Überbohrung der drei gesetzten Zieldrähte mit entsprechenden Hohlbohrern entsteht dann eine, im Querschnitt gesehen, relativ langerstreckte, grob rechteckförmige Bohrung.

Ist die Kanülierung nicht um 180° umfangswinkelversetzt, sondern um einen anderen Winkel, entstehen entsprechend abgeknickte Bohrungsprofile.

Sind mehrere umfangswinkelversetzte äußere Kanülierungen vorhanden, können auch andere geometrische Gebilde, wie "T"-förmige oder "t"-förmige Bohrungen erzielt werden. Die Handhabung ist für den Operateur einfach und sicher durchzuführen, denn er geht immer nach demselben Schema vor.

Dies beinhaltet Anzielen und Ansetzen der Vorrichtung in bekannter und gewohnter Weise, wie wenn nur ein einziger Zieldraht zum Anbringen nur einer einzigen kreisförmigen Bohrung bewerkstelligt werden soll. Durch das Vorsehen des zweiten Führungskanals kann zunächst unmittelbar benachbart und parallel zum ersten Zieldraht der zweite Zieldraht gesetzt werden. Nunmehr wird die abnehmbare Führungshülse so weit abgezogen, dass der zweite Zieldraht freiliegt, die Führungshülse aber noch auf dem ersten Zieldraht aufgeschoben ist. Durch Drehen der Führungshülse um den gewünschten Winkel um den ersten Zieldraht können dann eine dritte oder ggf. mehrere weitere Zieldrähte nach Wunsch gesetzt werden. Die jeweils zuvor durch den zweiten Bohrkanal gesetzten Zieldrähte sind dann in der oder den offenen Kanülierungen eingelegt.

Danach kann dann die Vorrichtung abgenommen werden und die im Knochen gesetzten und darin feststeckenden Zieldrähte mit entsprechenden Hohlbohrern überbohrt werden, um dann die gewünschte Bohrung mit dem gewünschten Querschnittsprofil im Knochen zu erstellen.

In einer Ausgestaltung der Erfindung ist eine einzige Kanülierung vorhanden, die um einen Winkel bis zu 180° winkelversetzt zum zweiten Führungskanal angeordnet ist.

Diese Maßnahme hat, wie zuvor erwähnt, den Vorteil, dass drei Zieldrähte unmittelbar benachbart gesetzt werden können, und zwar zwischen einer linearen Ausrichtung über eine Verbindungslinie der Mittelachsen der Zieldrähte gesehen bis zu einer abgewinkelten Ausrichtung.

In einer weiteren Ausgestaltung der Erfindung ist die einzige Kanülierung exakt um 180° winkelversetzt.

Diese Maßnahme hat den Vorteil, dass mit dieser Ausgestaltung eine exakt gerade ausgerichtete Bohrung mit rechteckförmigem Querschnitt bewerkstelligt werden kann.

In einer weiteren Ausgestaltung der Erfindung sind drei Kanülierungen vorhanden, die jeweils um 90° winkelversetzt zum zweiten Führungskanal angeordnet sind.

Diese Maßnahme hat den Vorteil, dass dadurch mehrere unterschiedliche Querschnitte von Bohrungen erzielt werden können.

Einmal ist es möglich, wenn nur die um 180° winkelversetzte Kanülierung belegt wird, wie zuvor erwähnt, die sich geradlinig erstreckende rechteckquerschnittsförmige Öffnung zu bewerkstelligen.

Wird eine Kanülierung, die nur um 90° winkelversetzt ist, belegt, wird eine entsprechend abgewinkelte Bohrung erzielt.

Werden zwei dieser Kanülierungen belegt, wird eine grob "T"-förmige Querschnittsstruktur erzielt.

Werden alle drei Kanülierungen belegt, kann ein "symmetrisch kreuzförmiges" Profil erzielt werden.

In einer weiteren Ausgestaltung der Erfindung weist die Führungshülse einen etwa stabförmigen Körper auf, in dem mittig der erste Führungskanal ausgespart ist, und der zweite Führungskanal ist an dem Körper angesetzt.

Diese Maßnahme hat den Vorteil, dass die beiden Führungskanäle fertigungstechnisch sehr einfach hergestellt werden können und dem Operateur zugleich durch den äußeren angesetzten zweiten Führungskanal ein Orientierungsmerkmal gegeben ist, aus dem er weiß, in welcher Lage und Orientierung der zweite Führungskanal zum ersten Führungskanal steht. So kann die Führungshülse aus einem stabförmigen Körper bestehen, der mittig eine Bohrung aufweist, die den ersten Führungskanal darstellt. An diesen Körper kann nun der zweite Führungskanal in Form eines Führungsrohres angefügt oder angesetzt werden. Dazu kann dieses Führungsrohr an die Außenseite oder in eine entsprechend eingeschnittene Vertiefung eingesetzt und darin befestigt werden, wobei dies von den jeweiligen Ausmaßen, insbesondere des Abstandes A, mitbestimmt wird.

In einer weiteren Ausgestaltung der Erfindung ist die zumindest eine Kanülierung als seitlich offene Längsnut am Körper der Führungshülse ausgebildet.

Diese Maßnahme hat den Vorteil, dass diese Nut einfach durch Ausfräsen des Körpers exakt maßgerecht und lagegerecht hergestellt werden kann. Das erleichtert auch die Handhabung dahingehend, dass nach Setzen der ersten beiden Zieldrähte und nach Abziehen der Führungshülse und Drehen um den längeren mittigen Zieldraht die Führungshülse lediglich wieder linear nach vorne geschoben werden muss, wobei dann der zweite Zieldraht in diese Längsnut eintritt. Dadurch können gewisse Fehlausrichtungen des zweiten Zieldrahtes in gewissem Maße toleriert werden. Der zweite Zieldraht wird ja quasi in der Kanülierung "geparkt", damit durch den zweiten Führungskanal wieder ein weiterer Führungsdraht entsprechend geführt eingebracht werden kann.

In einer weiteren Ausgestaltung der Erfindung ist in der Handhabe eine Öffnung ausgespart, die der Außenkontur der Führungshülse angepasst ist, so dass die Führungshülse nur in bestimmten Ausrichtungen in die Öffnung einschiebbar ist.

Auch diese Maßnahme erleichtert die Handhabung dahingehend, dass die Handhabungsperson die Führungshülse nach Abziehen und Drehen nur in bestimmte Drehstellungen wieder in die Öffnung einschieben kann, also den Drehstellungen, die dem gewünschten oder den gewünschten Drehwinkeln entsprechen.

In einer weiteren Ausgestaltung der Erfindung ist am distalen Ende des Armes eine Öffnung vorhanden, durch die eine durch den ersten Führungskanal durchgeschobener Zieldraht durchführbar ist.

Diese an sich bekannte Maßnahme hat den erheblichen Vorteil, dass die Vorrichtung an zwei gegenüberliegenden Stellungen des Knochens am ersten Zieldraht gehalten ist, nämlich zum einen über die Öffnung, durch die der Zieldraht durch das distale Ende des Armes hindurchreicht und gegenüberliegend noch durch die auf dem ersten Zieldraht aufliegende Führungshülse.

In einer weiteren Ausgestaltung der Erfindung sind am distalen Ende des Armes, benachbart zur Öffnung, Markierungen angebracht, die Zielpunkte der weiteren Zieldrähte, die durch den zweiten Führungskanal und die zumindest eine offene Kanülierung laufen, darstellen.

Diese Maßnahme hat den Vorteil, dass der Operateur nach Durchdringen des zweiten und der weiteren Zieldrähte anhand der Markierungen überprüfen kann, ob diese Zieldrähte exakt gesetzt sind.

In einer weiteren Ausgestaltung der Erfindung ist der erste Führungskanal als umfänglich geschlossener Führungskanal ausgebildet.

Diese Maßnahme hat den Vorteil, dass dadurch die abgezogene Führungshülse exakt geführt um den gesetzten ersten Zieldraht gedreht werden kann, ohne dass eine radiale Verschiebung oder Lageveränderung der Führungshülse möglich ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine Explosionsansicht eines ersten Ausführungsbeispiels einer erfindungsgemäßen Vorrichtung,
- Fig. 2: die Vorrichtung von Fig. 1 in zusammengebautem Zustand, wobei dargestellt ist, dass bereits ein erster Zieldraht durch die Führungshülse hindurchgeschoben ist,
- Fig. 3: eine der Fig. 2 entsprechende Darstellung, wobei dargestellt ist, dass ein zweiter Zieldraht durch den zweiten Führungskanal hindurchgeschoben ist,
- Fig. 4: eine Darstellung der Vorrichtung in einem Zustand während der Handhabung, bei der die Führungshülse vom zweiten Zieldraht abgezogen ist, jedoch noch auf dem ersten längeren Zieldraht aufsitzt,
- Fig. 5: eine der Fig. 4 entsprechende Darstellung, nachdem die Führungshülse um 180° um den ersten Zieldraht gedreht worden ist,
- Fig. 6: einen Zustand, nachdem die Führungshülse aus der Position von Fig. 5 wieder eingeschoben worden ist, der zweite Zieldraht in die erste Kanülierung eingetreten ist und bereits ein dritter Zieldraht durch den zweiten Führungskanal gesetzt ist,
- Fig. 7: einen Querschnitt etwa auf halber Länge durch die Führungshülse der Explosionsdarstellung von Fig. 1,
- Fig. 8: einen entsprechenden Querschnitt in dem Zustand von Fig. 2,
- Fig. 9: einen entsprechenden Querschnitt in dem in Fig. 3 dargestellten Bauzustand,
- Fig. 10: einen Querschnitt des Bauzustandes von Fig. 5, nachdem die Führungshülse wieder eingeschoben worden ist,
- Fig. 11: einen entsprechenden Querschnitt des Bauzustandes von Fig. 6,
- Fig. 12: eine Schnittdarstellung durch einen Knochen, in den die drei Zieldrähte gesetzt waren, nach Überbohren durch Hohlbohrer,
- Fig. 13: eine perspektivische Darstellung eines Unterschenkelknochens, in den die drei Zieldrähte gesetzt worden sind, also nach Abziehen der Vorrichtung aus dem Bauzustand von Fig. 6,
- Fig. 14: einen der Fig. 12 entsprechenden Zustand des Knochens nach Überbohren und Abziehen der drei Zieldrähte,
- Fig.: 15den Knochen von Fig. 14 nach einer möglichen Nachbearbeitung durch Knochenspatel zur Ausformung eines exakt rechteckigen Bohrungsquerschnittes,
- Fig.: 16 eine der Fig. 7 vergleichbare Querschnittsdarstellung eines zweiten Ausführungsbeispieles einer Führungshülse mit drei offenen Kanülierungen an der Außenseite,
- Fig.: 17 einen der Fig. 9 entsprechenden Bauzustand,

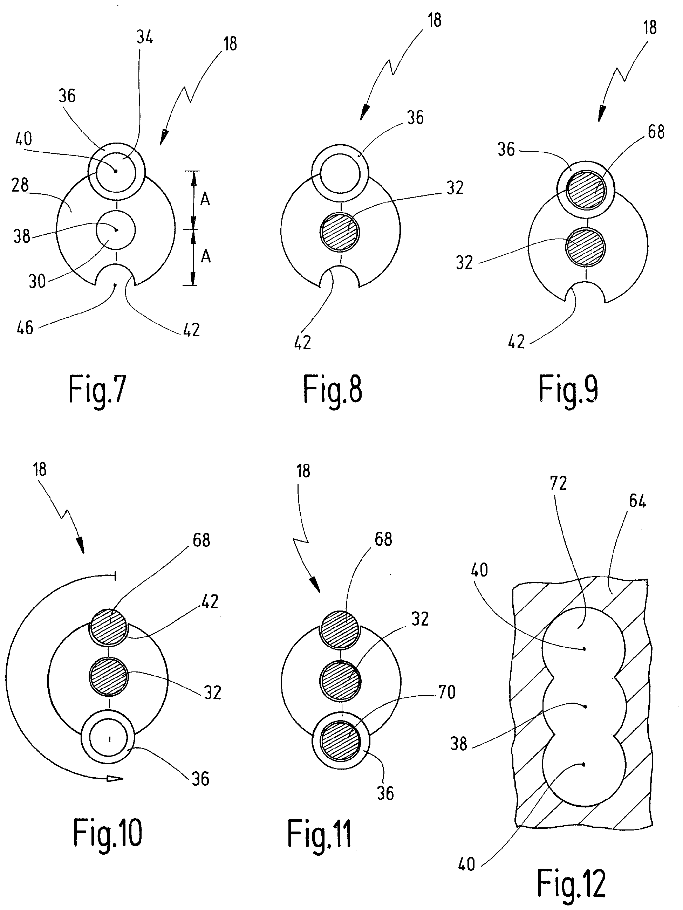

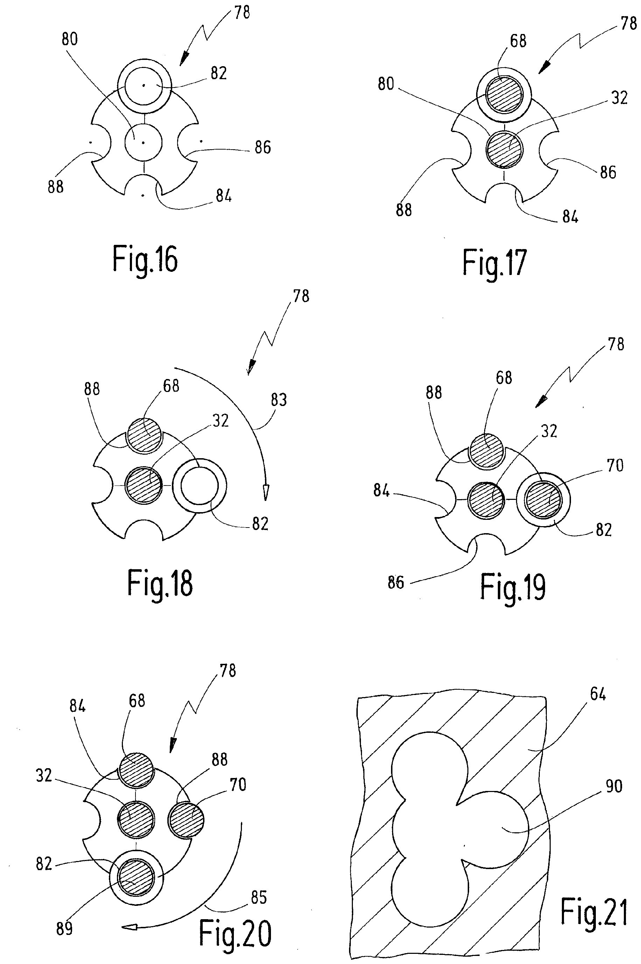

- Fig. 18: einen Bauzustand vergleichbar mit dem von Fig. 10, wobei hier allerdings die Führungshülse nur um 90° verdreht ist,
- Fig. 19: einen der Fig. 11 vergleichbaren Bauzustand mit drei gesetzten Zieldrähten,
- Fig. 20: einen Bauzustand nach einer weiteren Drehung um 90° mit vier gesetzten Zieldrähten, und
- Fig. 21: eine der Darstellung von Fig. 12 vergleichbare Darstellung mit einer Bohrung nach Überbohren der vier Zieldrähte von Fig. 20 zur Erzielung einer Öffnung mit "T"-Profil.

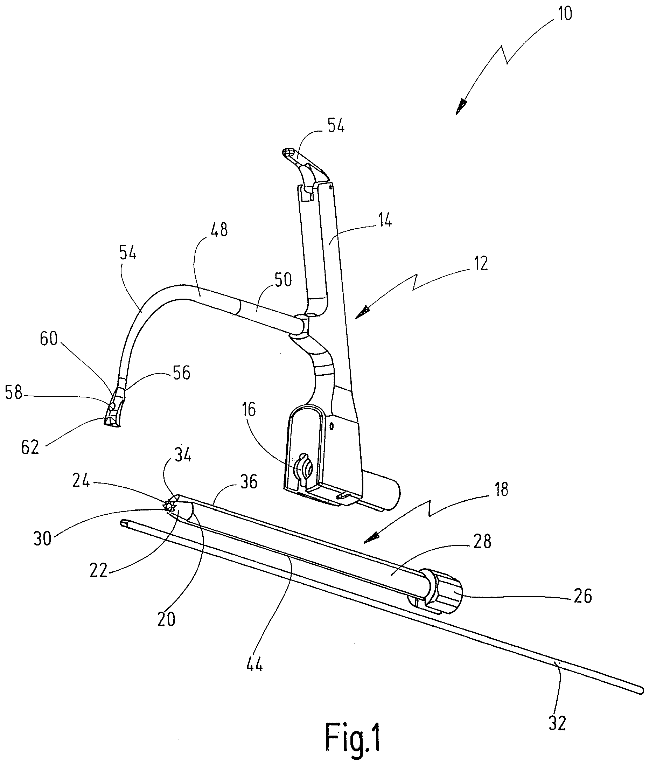

Ein in den Figuren 1 bis 11 dargestelltes erstes Ausführungsbeispiel einer erfindungsgemäßen Vorrichtung ist in seiner Gesamtheit mit der Bezugsziffer 10 bezeichnet.

Die Vorrichtung 10 weist eine Handhabe 12 auf, die einen etwa stabförmigen Griff 14 aufweist.

An einem Ende der Handhabe 12 ist eine Öffnung 16 vorgesehen, die zur Aufnahme einer Führungshülse 18 dient.

Die Führungshülse 18 ist, wie das insbesondere aus der Schnittdarstellung von Fig. 7 ersichtlich ist, grob in der Form eines Rohres 20 ausgebildet, dessen distales Ende 22 mit einem Zackenkranz 24 (Fig. 1) versehen ist. Am gegenüberliegenden Ende ist die Führungshülse 18 mit einer drehbaren Klemmhülse 26 versehen.

In dem eigentlichen Körper 28 der Führungshülse 18 ist ein erster mittiger durchgehender Führungskanal 30 vorhanden, der im vorliegenden Fall als eine durch den Körper 28 hindurchgehende mittige Bohrung ausgebildet ist. Der lichte Innendurchmesser des ersten Führungskanals 30 ist so, dass durch diesen passend ein erster Zieldraht 32, wie er in Fig. 1 dargestellt ist, hindurchgeschoben werden kann. Der Zieldraht 32 weist einen Außendurchmesser von 2,4 mm auf. Seitlich versetzt zum ersten Führungskanal 30 ist ein zweiter Führungskanal 34 vorhanden, der das Innere Lumen eines Führungsrohres 36 darstellt. Das Führungsrohr 36 ist in eine hier nicht näher bezeichnete Ausnehmung an der Außenseite des Körpers 28 der Führungshülse 18 eingesetzt und darin befestigt, bspw. verklebt oder verschweißt oder verlötet.

Aus der Schnittdarstellung von Fig. 7 ist ersichtlich, dass die Mittellängsachse 38 des ersten Führungskanals 30 parallel und in einem Abstand Azur Mittellängsachse 40 des zweiten Führungskanals 34 verläuft. Aus Fig. 1 und der Schnittdarstellung von Fig. 7 ist weiter zu entnehmen, dass an der Außenseite des Körpers 28 noch eine Kanülierung 42 vorhanden ist, und zwar in Form einer im Querschnitt halbkreisförmigen Längsnut 44.

Aus der Schnittdarstellung von Fig. 7 ist wieder zu erkennen, dass eine virtuelle Mittellängsachse 46 der Längsnut 44 ebenfalls in einem Abstand A zur Mittellängsachse 38 des ersten Führungskanals 30 liegt.

Außerdem liegt die Mittellängsachse 46 bzw. die Längsnut 44 genau diametral gegenüberliegend zum zweiten Führungskanal 34. In anderen Worten ausgedrückt, ist die Kanülierung 42 bzw. deren Mittellängsachse 46 um 180° umfangswinkelversetzt zum zweiten Führungskanal 34 bzw. dessen Mittellängsachse 40 angeordnet.

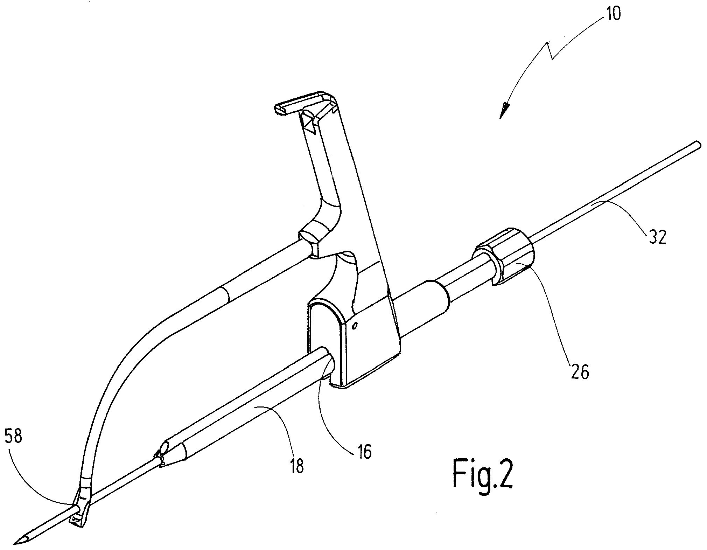

Zurückkehrend zu der Darstellung von Fig. 1 und 2 ist ersichtlich, dass von der Handhabe 12 im Abstand und etwa parallel zur Führungshülse 18 verlaufend ein Arm 48 vorspringt, der in einer Halterung 50 aufgenommen ist.

Ein Klemmhebel 54 hält den Arm 48 in der dargestellten Position, ein Drücken des Klemmhebels 54 erlaubt die Abnahme des Armes 48 und dieser kann entsprechend gereinigt oder durch einen neuen oder einen anders ausgeformten Arm ersetzt werden.

Der Arm 48 weist distal einen gebogenen Bereich 54 auf, dessen distales Ende 56 eine Öffnung 58 aufweist. Die Lage der Öffnung 58 ist derart, dass sie mit dem ersten Führungskanal 30 fluchtet, wenn die Führungshülse 18 in die Öffnung 16 an der Handhabe 12 eingeschoben ist, wie das insbesondere aus Fig. 2 ersichtlich ist.

Das bedeutet, wird ein erster Zieldraht 32 von proximal, also im Bereich der Klemmhülse 26, durch die Führungshülse 18 hindurchgeschoben, trifft dieser auf die Öffnung 58 und kann durch diese hindurchtreten.

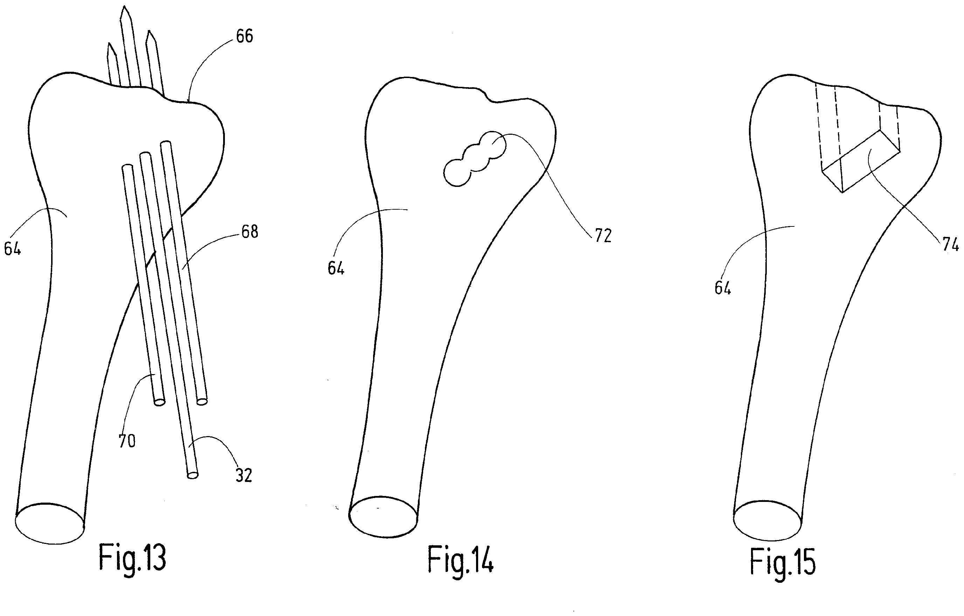

Die Vorrichtung 10 wird, wie das insbesondere in der DE 10 2007 057 075.0 A1 näher beschrieben und erläutert ist, seitlich geneigt an die Außenseite eines Knochens, bspw. an den in Fig. 13 dargestellten Unterschenkelknochen 64, angesetzt. Dabei erleichtert der Zackenkranz 24 den Ansatz und das Halten der Führungshülse 18 an dieser Stelle des Knochens 64 bzw. an der diesen Knochen bedeckenden Haut.

Das distale Ende 56 des gebogenen Armes 48 wird dabei in das geöffnete Kniegelenk eingebracht und so am Tibiaplateau 60 positioniert, dass ein durch den Knochen 64 durchgeschobener erster Zieldraht 32, wie das in Fig. 13 ersichtlich ist, durch das Tibiaplateau 60 durchtritt und durch die Öffnung 58 hindurchgeführt ist.

Der Übersicht halber ist in Fig. 2 der Knochen nicht dargestellt, da dieser erste Arbeitsschritt an sich aus der zuvor erwähnten DE 10 2007 057 075.0 A1 bekannt ist.

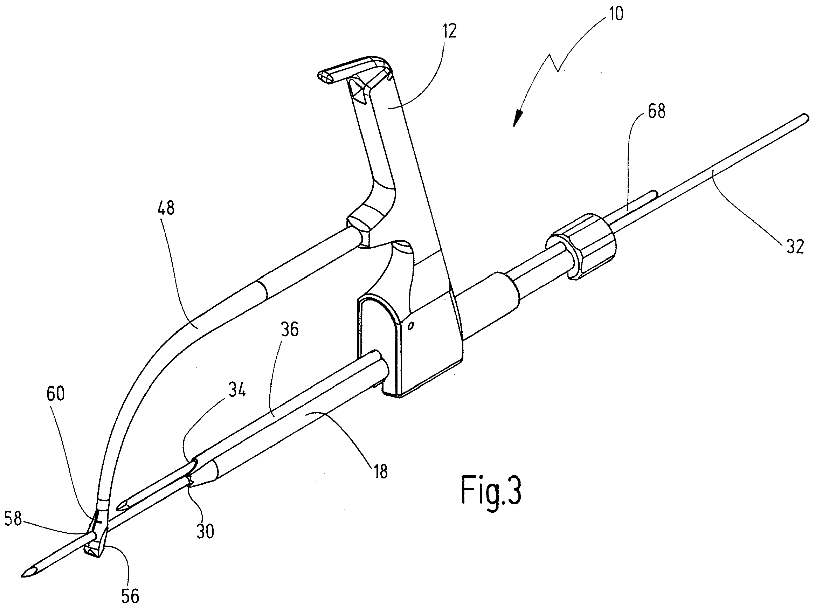

Die Handhabung und das Setzen der weiteren Zieldrähte soll nun anhand der Bildfolge von Fig. 3 bis Fig. 6 und den Schnittdarstellungen von Fig. 8 bis Fig. 11 näher beschrieben und erläutert werden.

Nach Setzen des ersten Zieldrahtes 32, wie das in Fig. 2 dargestellt ist, wird durch den zweiten Führungskanal 34 ein weiterer bzw. zweiter Zieldraht 68 gesetzt, wie das in Fig. 3 bzw. Fig. 9 ersichtlich ist.

Es ist ersichtlich, dass der erste Zieldraht 32 wesentlich länger als der weitere zweite Zieldraht 68 ist. In seiner maximalen Vorschubstellung gelangt die hier nicht näher dargestellte distale Spitze des zweiten Zieldrahtes 68 bis an die Markierung 60 am distalen Ende 56 des gekrümmten Armes 48 heran. Hat er diese Stelle erreicht, ist das ein Hinweis für den Operateur, dass der zweite weitere Zieldraht 68 lage- und zielgerecht gesetzt worden ist.

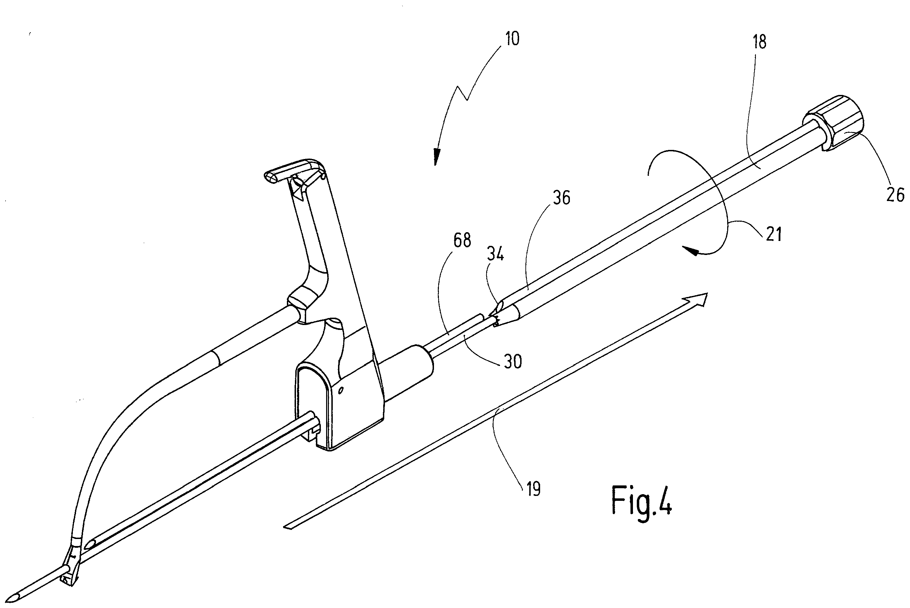

Nunmehr wird die Klemmhülse 26 verdreht, so dass die Führungshülse 18 so weit nach proximal abgezogen werden kann (siehe Pfeil 19), bis der zweite weitere Zieldraht 68 aus dem zweiten Führungskanal 34 ausgetreten ist, wobei dieser Bauzustand in Fig. 4 dargestellt ist. Die Länge des Abzugsweges soll durch den Pfeil 19 repräsentiert werden.

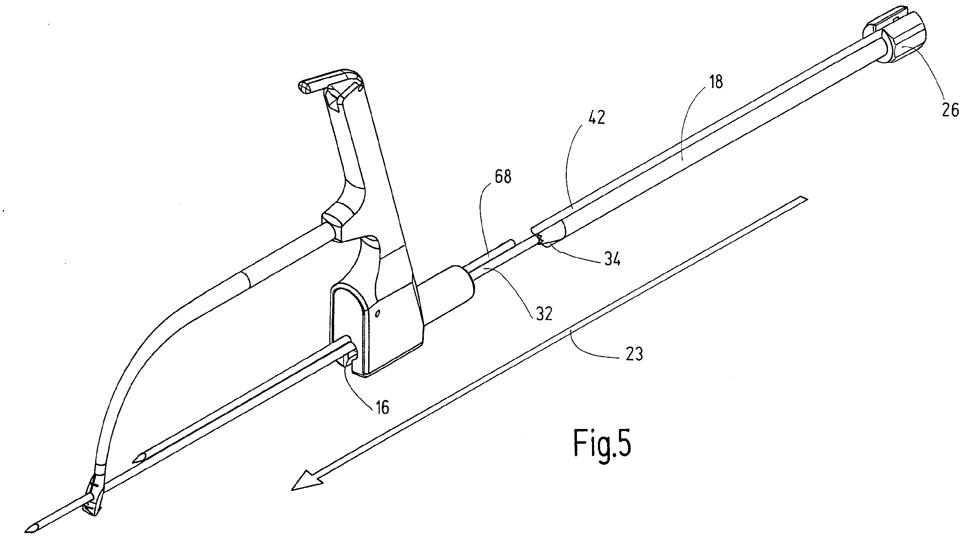

Nunmehr ist es möglich, die Führungshülse 18 um den ersten Zieldraht 32 zu drehen, wie das in Fig. 4 durch den Pfeil 21 dargestellt ist. Die Drehung wird um 180° durchgeführt, so dass dann die Kanülierung 42 an der Außenseite der Führungshülse 18 in einer Ausrichtung liegt, in der bei Vorschieben der Führungshülse 18 der zweite Zieldraht 68 in die Kanülierung 42 eintreten kann. Diese Situation ist in Fig. 5 dargestellt.

Wird nun die Führungshülse 18 entsprechend dem Pfeil 32 nach distal verschoben, tritt der Zieldraht 68 in die Kanülierung 42 ein (siehe Fig. 10). Anschließend wird die Führungshülse 18 durch die Öffnung 16 hindurchgeschoben, wobei diese so ausgestaltet ist, dass die Führungshülse 18 entweder in dieser oder in der um 180° verdrehten Stellung durchgeschoben werden kann. Nach Feststellen durch die Klemmhülse 26 wird dann durch den in der Darstellung von Fig. 5 an der Unterseite liegenden zweiten Führungskanal 34 bzw. durch das Führungsrohr 36 ein dritter Zieldraht 70 hindurchgeschoben.

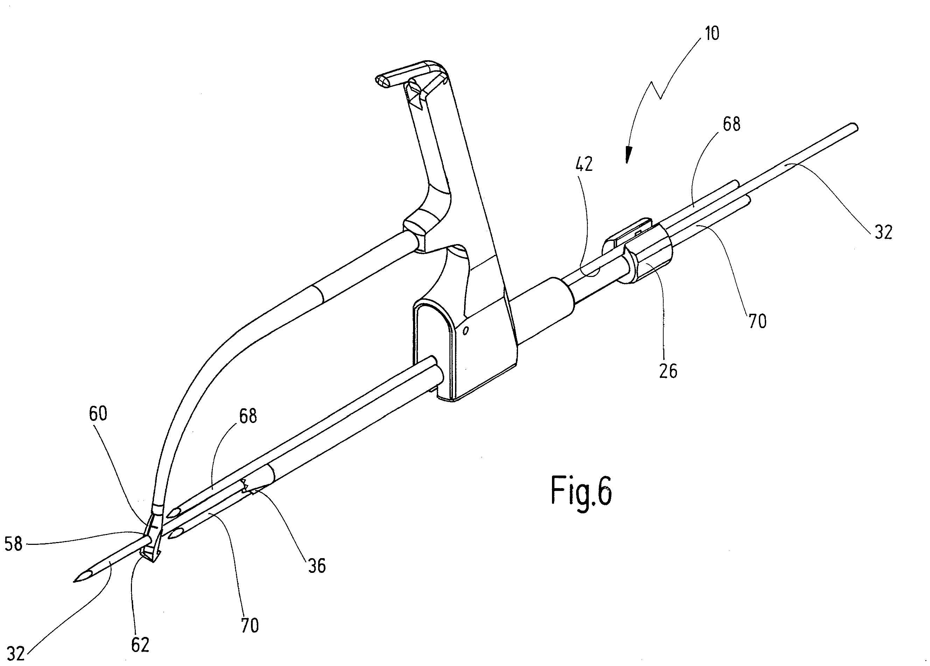

Diese Situation ist in Fig. 6 und in Fig. 11 dargestellt.

Auch der dritte Zieldraht 70 kann so weit vorgeschoben werden, bis er die entsprechende Markierung 62 am distalen Ende 56 des Armes 48 erreicht hat, so dass wieder der Operateur überprüfen kann, ob auch dieser dritte Zieldraht 70 an der zutreffenden Stelle aus dem Tibiaplateau 66 hervorragt.

Anschließend wird die Führungshülse 18 abgezogen, der Arm 48 von der Handhabe 12 gelöst, so dass die gesamte Vorrichtung 10 von den drei gesetzten Zieldrähten 32, 68 und 70 abgenommen ist.

Diese Situation ist in Fig. 13 dargestellt, das heißt, in den Unterschenkelknochen 64 wurden von "unten" mittels der Vorrichtung 10 die drei Zieldrähte 32, 68, und 70 so durchgeschoben, dass sie alle drei parallel zueinander ausgerichtet vom Tibiaplateau 66 hochstehen.

Der Abstand A zwischen den drei Mittellängsachsen der drei Zieldrähte 32, 68 und 70 ist so gewählt, dass diese mit einem 4,5-mm-Bohrer überbohrt werden können. Dabei werden zunächst die beiden äußeren Zieldrähte 68 und 70 überbohrt und abschlie-ßend wird der mittlere Zieldraht 32 ebenfalls überbohrt.

Daraus resultiert dann eine Bohrung 72, wie sie in Fig. 12 und 14 dargestellt ist.

Soll ein exakt rechteckförmiger Kanal 74, wie er in Fig. 15 dargestellt worden ist, erzielt werden, kann noch in die Bohrung 72 ein entsprechend rechteckförmiger Dilatator eingeschoben werden, um die im Überschneidungsbereich verbliebenen hochstehenden Knochenbereiche abzulösen.

In den Kanal 74 kann nun bspw. ein rechteckförmiges Sehnentransplantat bei der Rekonstruktion eines vorderen Kreuzbandes pass- und formgerecht eingesetzt und verankert werden.

In den Figuren 16 bis 21 ist ein zweites Ausführungsbeispiel einer erfindungsgemäßen Vorrichtung dargestellt, wobei der Unterschied lediglich darin besteht, dass die Führungshülse 78 und entsprechend die Öffnung, in die diese in die Handhabe 12 eingeschoben werden kann, anders ausgebildet sind.

Aus der Schnittdarstellung von Fig. 16, die der Schnittdarstellung von Fig. 7 entspricht, ist zu erkennen, dass auch hier wieder ein mittiger erster Führungskanal 80 vorhanden ist. In dem zuvor beschriebenen Abstand A und parallel dazu verlaufend ist ein zweiter Führungskanal 82 vorhanden. Auch hier ist eine erste Kanülierung 84 in der Außenseite der Führungshülse 78 vorhanden, deren Mittellängsachse wieder parallel und im selben Abstand A zur Mittellängsachse des ersten Führungskanals 80 verläuft, wie das zuvor im Zusammenhang mit dem ersten Ausführungsbeispiel beschrieben worden ist.

In Abweichung zum ersten Ausführungsbeispiel sind beim zweiten Ausführungsbeispiel an der Außenseite der Führungshülse 78 noch eine zweite und eine dritte Kanülierung 86 bzw. 88 ausgespart. Auch diese bestehen wieder in Form von längs verlaufenden Nuten mit etwa halbkreisförmigem Querschnitt und deren Mittellängsachsen stehen wieder im Abstand A zu der Mittellängsachse des ersten Führungskanals 80.

Mit dem zweiten Ausführungsbeispiel können, wie zuvor beschrieben, drei parallel zueinander verlaufende, in einer Reihe ausgerichtete Zieldrähte gesetzt werden.

Zusätzlich ist es aber auch möglich, nun andere Geometrien an resultierenden Bohrungen zu erzielen.

Fig. 17 entspricht einem Bauzustand, wie er in Fig. 9 beschrieben ist, also der Zustand, nachdem die ersten beiden Zieldrähte 32 und 68 gesetzt sind.

Wie aus dem Übergang von Fig. 17 zu Fig. 18 ersichtlich, wird nach Abziehen der Führungshülse 78 vom zweiten Zieldraht 68 diese nur um 90° verdreht, wie das durch einen Pfeil 83 angedeutet ist. Anschließend wird die Führungshülse 78 wieder axial eingeschoben.

Dadurch tritt dann der zweite Zieldraht 68 in die um 90° entgegen dem Uhrzeigersinn winkelversetzte vierte Kanülierung 88 ein. Nunmehr kann durch den zweiten Führungskanal 82 ein dritter Zieldraht 70 eingeschoben werden, so dass sich die aus Fig. 19 resultierende Ausrichtung der drei Zieldrähte 32, 68, 70 an den Ecken eines Dreiecks ergibt.

Durch erneutes Lösen und Abziehen der Führungshülse 78 vom dritten Zieldraht 70 kann die Führungshülse 78 erneut um 90° gedreht werden, wie das in Fig. 20 durch einen Pfeil 85 dargestellt ist.

Der dritte Zieldraht 70 ist nunmehr in die dritte Kanülierung 88 eingetreten, der zweite Zieldraht 68 liegt in der ersten Kanülierung 84. Nunmehr kann in den wieder freigewordenen zweiten Führungskanal 82 ein vierter Zieldraht 89 gesetzt werden.

Nach Abnehmen der Vorrichtung und Überbohren der vier Zieldrähte 32, 68, 70 und 89 resultiert dann im Knochen 64 eine Bohrung mit einem grob "T"-förmigem Profil, wie das in Fig. 21 dargestellt ist.

## Patentansprüche

1. Vorrichtung zum Anzielen und Einbringen von mehreren Führungskanälen in einen Knochen (64), mit einer Handhabe (12), in der abnehmbar eine einzige Führungshülse (18, 78) aufgenommen ist, in der ein mittiger erster Führungskanal (30, 80) für einen ersten Zieldraht (32) vorhanden ist, wobei die Führungshülse (18, 78) über deren distales Ende (22) an einen Knochen (64) anlegbar ist, und mit einem von der Handhabe (12) vorstehenden Arm (48), dessen distales Ende (56) an einer weiteren Stelle am Knochen (64) anlegbar ist, wobei die Führungshülse (18, 78) einen zweiten Führungskanal (34) für einen weiteren Zieldraht (68) aufweist, und eine Mittellängsachse (40) des zweiten Führungskanals (34) in einem Abstand (A) zur Mittellängsachse (38) des ersten Führungskanals (30, 80) und außerdem parallel zu diesem verläuft, **dadurch gekennzeichnet, dass** an der Führungshülse (18, 78) zumindest eine Kanülierung (42; 84, 86, 88) vorhanden ist, deren Mittellängsachse (46) im gleichen Abstand (A) und parallel zur Mittelachse (38) des ersten Führungskanals (30, 80) verläuft, und dass die zumindest eine Kanülierung (42; 84, 86, 88) umfangswinkelversetzt zur Mittellängsachse (40) des zweiten Kanals (34) verläuft, so dass ein durch den zweiten Führungskanal (34) eingebrachter weiterer Zieldraht (68) durch Abziehen, Drehen um den ersten Zieldraht (32) und wieder Einsetzen der Führungshülse (18, 30) in die zumindest eine Kanülierung (42; 84, 86, 88) einbringbar ist.

2. Vorrichtung nach Anspruch 1, **dadurch gekennzeichnet, dass** eine einzige Kanülierung (42) vorhanden ist, die um einen Winkel bis zu 180° winkelversetzt zum zweiten Führungskanal (34) angeordnet ist.

3. Vorrichtung nach Anspruch 1 oder 2, **dadurch gekennzeichnet, dass** eine einzige Kanülierung (42) vorhanden ist, die um 180° winkelversetzt zum zweiten Führungskanal (34) angeordnet ist.

4. Vorrichtung nach Anspruch 1, **dadurch gekennzeichnet, dass** drei Kanülierungen (84, 86, 88) vorhanden sind, die jeweils um 90° winkelversetzt zum zweiten Führungskanal (34) angeordnet sind.

5. Vorrichtung nach einem der Ansprüche 1 bis 4, **dadurch gekennzeichnet, dass** die Führungshülse (18, 78) einen etwa stabförmigen Körper (28) aufweist, in dem mittig der erste Führungskanal (30, 80) ausgespart ist, und dass der zweite Führungskanal (84, 82) an dem Körper (28) angesetzt ist.

6. Vorrichtung nach einem der Ansprüche 1 bis 5, **dadurch gekennzeichnet, dass** die zumindest eine Kanülierung (42; 84, 86, 88) als seitlich offene Längsnut (44) am Körper (28) der Führungshülse (18, 78) ausgebildet ist.

7. Vorrichtung nach einem der Ansprüche 1 bis 6, **dadurch gekennzeichnet, dass** in der Handhabe (12) eine Öffnung (16) ausgespart ist, die der Außenkontur der Führungshülse (18, 78) angepasst ist, so dass die Führungshülse (18, 78) nur in bestimmten Ausrichtungen in die Öffnung (16) einschiebbar ist.

8. Vorrichtung nach einem der Ansprüche 1 bis 7, **dadurch gekennzeichnet, dass** am distalen Ende (56) des Armes (48) eine Öffnung (58) vorhanden ist, durch die ein durch den ersten Führungskanal (30, 80) durchgeschobener erster Zieldraht (32) durchführbar ist.

9. Vorrichtung nach Anspruch 8, **dadurch gekennzeichnet, dass** am distalen Ende des Armes (48), benachbart zur Öffnung (58), Markierungen (60, 62) angebracht sind, die Zielpunkte der weiteren Zieldrähte (68, 70 89), die durch den zweiten Führungskanal (34, 82) und durch die zumindest eine offene Kanülierung (42,; 84, 86, 88) laufen, darstellen.

10. Vorrichtung nach einem der Ansprüche 1 bis 9, **dadurch gekennzeichnet, dass** der erste Führungskanal (30, 80) als umfänglich geschlossener Führungskanal ausgebildet ist.

## Claims

1. Device for lining up and introducing a plurality of guiding channels in a bone (64), comprising a handle (12) in which there is removably held a single guiding sleeve (18, 78), in which a central, first guiding channel (30, 80) for a first aiming wire (32) is present, the guiding sleeve (18, 78) being able to be placed with its distal end (22) on a bone (64), and comprising an arm (48) which projects from the handle (12) and the distal end (56) of which can be placed on the bone (64) at another site, wherein the guiding sleeve (18, 78) has a second guiding channel (34) for a further aiming wire (68), wherein a central longitudinal axis (40) of the second guiding channel (34) runs at a distance (A) from the central longitudinal axis (38) of the first guiding channel (30, 80) and also running parallel thereto, **characterized in that** at the guiding sleeve (18, 78) there is at least one cannulation (42; 84, 86, 88), the central longitudinal axis (46) of which runs at the same distance (A) from and parallel to the central axis (38) of the first guiding channel (30, 80), and that the at least one cannulation (42; 84, 86, 88) runs offset in relation to the central longitudinal axis (40) of the second channel (34) in terms of the circumferential angle, so that a further aiming wire (68) introduced through the second guiding channel (34) can be introduced into the at least one open cannulation (42; 84, 86, 88) by pulling off the guiding sleeve (18, 30), turning it about the first aiming wire (32) and reinserting it.

2. Device according to Claim 3, **characterized in that** there is a single cannulation (42), which is arranged angularly offset in relation to the second guiding channel (34) by an angle of up to 180°.

3. Device according to Claim 1 or 2, **characterized in that** there is a single cannulation (42), which is arranged angularly offset in relation to the second guiding channel (34) by 180°.

4. Device according to Claim 1, **characterized in that** there are three cannulations (84, 86, 88), which are arranged angularly offset in relation to the second guiding channel (34) in each case by 90°.

5. Device according to one of Claims 1 to 4, **characterized in that** the guiding sleeve (18, 78) has an approximately bar-shaped body (28), in which the first guiding channel (30, 80) is centrally cut out, and **in that** the second guiding channel (84, 82) is set on the body (28).

6. Device according to one of Claims 1 to 5, **characterized in that** the at least one open cannulation (42; 84, 86, 88) is formed as a laterally open longitudinal groove (44) on the body (28) of the guiding sleeve (18, 78).

7. Device according to one of Claims 1 to 6, **characterized in that** cut out in the handle (12) is an opening (16), which is adapted to the outer contour of the guiding sleeve (18, 78) such that the guiding sleeve (18, 78) can only be pushed into the opening (16) in certain orientations.

8. Device according to one of Claims 1 to 7, **characterized in that** at the distal end (56) of the arm (48) there is an opening (58) through which an aiming wire (32) pushed through the first guiding channel (30, 80) can be passed.

9. Device according to Claim 8, **characterized in that** markings (60, 62) are provided at the distal end of the arm (48), adjacent to the opening (58), representing target points of the further aiming wires (68, 70, 89), which run through the second guiding channel (34, 82) and through the at least one open cannulation (42; 84, 86, 88).

10. Device according to one of Claims 1 to 9, **characterized in that** the first guiding channel (30, 80) is formed as a circumferentially closed guiding channel.

## Revendications

1. Ensemble de visée et de formation de plusieurs canaux de guidage dans un os (64), l'ensemble présentant
une poignée (12) dans laquelle une unique douille de guidage (18, 78) dans laquelle un premier canal central de guidage (30, 80) est prévu pour un premier fil de visée (32) est reprise de manière libérable, la douille de guidage (18, 78) pouvant être placée par une extrémité distale (22) sur un os (64) et
un bras (48) débordant de la poignée (42) et dont l'extrémité distale (56) peut être placée sur l'os (64) en un autre emplacement,
la douille de guidage (18, 78) présentant un deuxième canal de guidage (34) pour un deuxième fil de visée (68), et
un axe longitudinal central (42) du deuxième canal de guidage (34) s'étendant à une distance (A) de l'axe longitudinal central (38) du premier canal de guidage (30, 80) et s'étendant en outre parallèlement à ce dernier,
**caractérisé en ce que**
au moins une canulation (42; 84, 86, 88) dont l'axe longitudinal central (46) s'étend à la même distance (A) et parallèlement à l'axe central (38) du premier canal de guidage (30, 80) est prévue sur la douille de guidage (18, 78) et
**en ce que** la ou les canulations (42; 84, 86, 88) s'étendent à un décalage angulaire périphérique par rapport à l'axe longitudinal central (40) du deuxième canal (34) de telle sorte qu'un autre fil de visée (68) passé dans le deuxième canal de guidage (34) peut être inséré dans la ou les canulations (42; 84, 86, 88) par rétraction, rotation autour du premier fil de visée (32) et réinsertion de la douille de guidage (18, 30).

2. Ensemble selon la revendication 1, **caractérisé en ce qu'**il présente une seule canulation (42) disposée à un décalage angulaire d'un angle pouvant atteindre 180° par rapport au deuxième canal de guidage (34).

3. Ensemble selon les revendications 1 ou 2, **caractérisé en ce qu'**il présente une seule canulation (42) disposée à un décalage angulaire d'un angle pouvant atteindre 180° par rapport au deuxième canal de guidage (34).

4. Ensemble selon la revendication 1, **caractérisé en ce qu'**il présente trois canulations (84, 86, 88) décalées chacune d'un angle de 90° par rapport au deuxième canal de guidage (34).

5. Ensemble selon l'une des revendications 1 à 4, **caractérisé en ce que** la douille de guidage (18, 78) présente un corps (28) sensiblement en forme de barreau au milieu duquel le premier canal de guidage (30, 80) est découpé et **en ce que** le deuxième canal de guidage (84, 82) est placé sur le corps (28).

6. Ensemble selon l'une des revendications 1 à 5, **caractérisé en ce que** la ou les canulations (42; 84, 86, 88) sont configurées comme rainures longitudinales (44) ouvertes latéralement prévues sur le corps (28) de la douille de guidage (18, 78).

7. Ensemble selon l'une des revendications 1 à 6, **caractérisé en ce qu'**une ouverture qui est adaptée au contour extérieur de la douille de guidage (18, 78) est ménagée dans la poignée (12) de telle sorte que la douille de guidage (18, 78) ne puisse être insérée dans l'ouverture (16) que dans des orientations définies.

8. Ensemble selon l'une des revendications 1 à 7, **caractérisé en ce qu'**une ouverture (58) par laquelle un premier fil de visée (32) passé dans le premier canal de guidage (30, 80) peut être passé est prévue sur l'extrémité distale (56) du bras (48).

9. Ensemble selon la revendication 8, **caractérisé en ce qu'**à l'extrémité distale du bras (48) voisine de l'ouverture (58), des repères (60, 62) sont ménagés et représentent les points de visée des autres fils de visée (68, 70, 89) qui s'étendent dans le deuxième canal de guidage (34, 82) et dans la ou les canulations ouvertes (42; 84, 86, 88).

10. Ensemble selon l'une des revendications 1 à 9, **caractérisé en ce que** le premier canal de guidage (30, 80) est configuré comme canal de guidage à périphérie fermée.
